Europäisches Patentamt

(19) European Patent Office

Office européen des brevets

(11) Publication number : **0 397 413 B1**

## (12) EUROPEAN PATENT SPECIFICATION

(45) Date of publication of patent specification :
12.01.94 Bulletin 94/02

(51) Int. Cl.⁵ : **C12N 5/02, C12N 5/10**

(21) Application number : **90304872.6**

(22) Date of filing : **04.05.90**

(54) **Improved particle gun.**

(30) Priority : **12.05.89 US 351075**

(43) Date of publication of application :
**14.11.90 Bulletin 90/46**

(45) Publication of the grant of the patent :
**12.01.94 Bulletin 94/02**

(84) Designated Contracting States :
**AT BE CH DE DK ES FR GB GR IT LI LU NL SE**

(56) References cited :
**EP-A- 0 331 855
PARTICULATE SCIENCE AND TECHNOLOGY,
vol. 5, no. 1, 1987, New York, NY (US); J.C.
SANFORD et al., pp. 27-37**

(73) Proprietor : **PIONEER HI-BRED
INTERNATIONAL, INC.
700 Capital Square 400 Locust Street
Des Moines Iowa 50309 (US)**

(72) Inventor : **Tomes, Dwight
8 Beechwood
Cumming, IA 50061 (US)**

(74) Representative : **Goldin, Douglas Michael et al
J.A. KEMP & CO. 14, South Square Gray's Inn
London WC1R 5EU (GB)**

EP 0 397 413 B1

## Description

BACKGROUND OF THE INVENTION

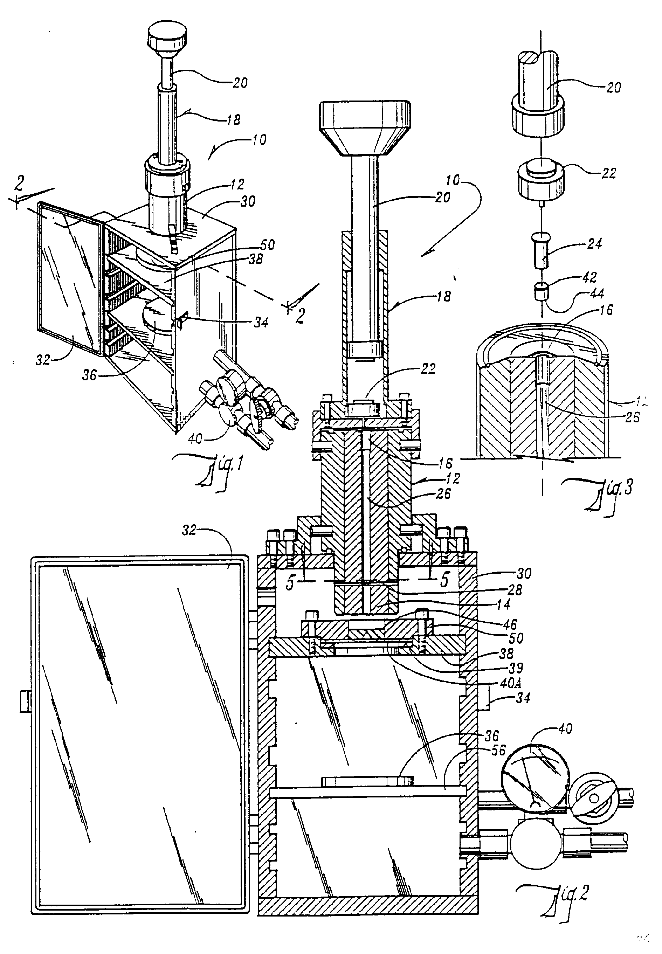

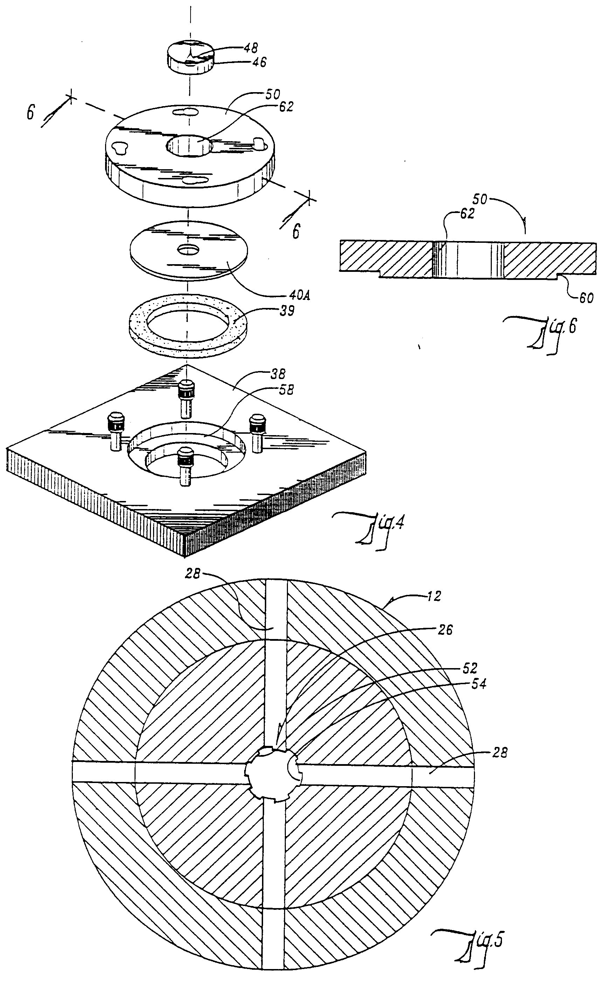

This invention relates to an improved particle gun for transferring biological materials such as nucleic acids into the cytoplasm of living cells. With the rapid advancement of recombinant DNA technology, there is a wide-ranging need for biologists to transfer biologic substances from one cell to another, and to transfer synthetic biological material into living cells to exert their activity therein. Such materials can include biological stains, proteins (antibodies or enzymes), and, most commonly, nucleic acids genetic material (either RNA or DNA). Most of the techniques used are painstakingly slow and use methods which transport materials into, at most, only a few cells at a time. More recently, there has been developed a particle bombardment process which utilizes a particle gun, as described in Sanford, et al, 1987, "Delivery of Substances Into Cells And Tissues Using A Particle Bombardment Process," Journal of Particle Science and Technology 5:27-37. The basic particle gun disclosed in this article is illustrated at Figure 3 of page 33 of the article. There it can be seen that the particle gun comprises a firing pin, barrel, a macroprojectile and a stopping plate, surrounded by a vacuum chamber. The barrel of the Sanford et al. gun has a smooth bore. In using a particle gun to transfer biological material into the cytoplasm of living target cells, it is important to avoid carrying unwanted debris into the cells which might result in damage or death to a large number of cells. Debris is undesirable because it may interfere with the success of the procedure; in fact it may cause the target cells to die. Unwanted cellular damage referred to as "blow by" results from debris that occurs when the macroprojectile is too small for the smooth bore barrel, or may result when the macroprojectile or stopping plate materials fail. Failure of the macroprojectile results in small particles of the ultra high molecular weight polyethylene (UHMWPE) material typically used extruding through the stopping plate at a high velocity. Stopping plate failure results when small pieces of the polycarbonate stopping plate are released from the plate upon impact by the macroprojectile. Any one of these sources of debris can substantially lower efficiency and increase unwanted cellular damage or death. The prior art particle gun has a vent near the forward muzzle end of the barrel. However, this venting, even when used with an associated vacuum, is unsuccessful in preventing blow by.

Another problem with the earlier-described Sanford et al. particle gun is poor tolerance for macroprojectile size variations within the range normally obtained by conventional manufacturing methods, whether machining or extrusion. Since the macroprojectiles manufactured from UHMWPE vary in size by as much as 0.0508mm (0.002 inches) as they are delivered, many of these must be discarded.

Accordingly, one objective of the present invention is to provide an improved particle gun which produces an increased number of transformed cells while at the same time lessening the risk of cell damage or death. Another objective of the present invention is to further increase the number of transformed cells by employing inert high mass metallic beads as microprojectiles in combination with certain preferred polyamine adjuvant materials.

The method and means of accomplishing each of these objectives will become apparent from the detailed description of the invention which follows hereinafter.

BRIEF DESCRIPTION OF THE DRAWINGS

Figure 1 is a perspective view of the improved particle gun of this invention.
Figure 2 shows an elevated front view in partial section along line 2-2 of Figure 1.
Figure 3 shows an exploded view of the rifled barrel, the firing pin, cartridge and macroprojectile.
Figure 4 shows an exploded view of the stopping plate and stopping plate holder assembly.
Figure 5 is a sectional view taken along line 5-5 of Figure 2 and shows the rifled barrel.
Figure 6 is a sectional view of the stopping plate holder taken along line 6-6 of Figure 4.

SUMMARY OF THE INVENTION

This invention relates to a particle gun for transporting biological materials such as DNA and RNA into the cytoplasm or nuclei of living intact target cells. The improvement in the particle gun primarily rests in rifling the bore of the gun barrel, rather than leaving it smooth as in the prior art. The result is a lessening of failures, a lessening of the risk of blow by damage to the target cells, and an improved transformation or material transfer rate. The invention also relates to an improved macroprojectile and to a method of transport of biological materials by an improved bombardment process as well as to a method of preparing microprojectiles for use in the process.

DETAILED DESCRIPTION OF THE INVENTION

Figures 1 and 2 illustrate the improved particle gun of this invention. The basic components of the gun, referred to generally at 10, are the barrel 12 having a muzzle end 14 and a chamber end 16. A removable hand firing bolt apparatus is depicted at 18 and generally comprises a downwardly removable ram 20 which strikes on firing pin 22. The entire bolt apparatus 18 may be removed by a simple twist so that a new cartridge 24 may be placed in chamber 16. The firing pin apparatus 22 is of conventional construction for 5.59mm (.22 cal.) rimfire cartridges and will not be further described herein.

The bore 26 of barrel 12 has a vent 28 near its muzzle end 14. An enclosed stainless steel chamber box 30 surrounds muzzle end 14. It has a hinged door 32 which may be unlatched and opened or closed via a latch 34. Inside of enclosure chamber 30 is a petri dish 36, a sample tray 56 and a stopping plate holder shelf 38. Petri dish 36 rests on sample tray 56 in such position that target cells in the petri dish are located on the axis of barrel 26. Sample tray 56 and stopping plate holder shelf 38 are slidably removable from the chamber 30. Chamber 30 is in communication with a vacuum means designated at 40.

The particle gun is loaded for use to transport biological material in the following manner. A macroprojectile 42 is inserted into the chamber 16 and pushed downwardly into the bore 26. The forward end 44 of the macroprojectile carries a small amount of biological material (DNA, for example) mixed with a carrier. The carrier generally comprises a substantially inert metal in the form of small beads which function as microprojectiles. Generally the microprojectiles have diameters within the range of from about 1 μm to about 4 μm. These beads can be made from tungsten, palladium, iridium, platinum or gold. Tungsten is preferred. The most preferred bead diameter is from about 1 μm to about 1.5 μm.

In the most preferred process, the beads are mixed with a small amount of biological material such as DNA or RNA. This is mixed with calcium chloride and a certain amount of polyamine is added.

Generally the ranges of each of these ingredients should be as follows:

Twenty-five μl of tungsten particles at a concentration of 50-200mg in 2ml sterile water are placed in a sterile 10 ml centrifuge tube and agitated to suspend the beads. The preferred amount of beads is 100mg/2ml. Twenty-five ul of the suspended tungsten beads are placed in an Eppendorf tube and DNA is added at a concentration of 1 μg/ul with the amount varying between 1μl and 20μl, the preferred amount being 10ml. A calcium chloride solution of 25μl and having a concentration between 1.0-4.0M, preferably 2.5M is mixed with the DNA/bead mixture.

While addition of spermidine to the biological material/microprojectile combination has been previously employed, it has now been more broadly discovered that addition of a variety of polyamines to mixtures of tungsten beads and DNA or RNA in preparing microprojectiles significantly improves rates of transformation. While not intending to be limited by theory, it is believed that the polyamine improves delivery of biological material to the cells in this process by improving adherence of the materials to the microprojectile beads. Suitable polyamines have been found to include, for example, spermine, spermidine, caldine, thermine. The preferred polyamine, spermine, has been found to be superior to the previously disclosed spermidine additive. Accordingly, at this point a polyamine, preferably spermine, in an amount of 10μl and a concentration between 0.05M and 0.5M, preferably at 0.1M is added followed by finger vortexing. This mixture is allowed to stand for 10 minutes prior to centrifugation for one to two minutes at 9,000 rpm. The microprojectile mixture forms a pellet at the bottom of the Eppendorf tube. Before use, supernatant is withdrawn from the tube to provide a final volume of 30 μl.

The DNA/bead mixture is sonicated briefly to suspend the microprojectiles prior to use. The suspended microprojectiles carrying the biological material are transferred to the forward end of the macroprojectile by micropipette in aliquots of 1-5μl, with 1.5μl preferred.

The macroprojectile 42 is placed into the barrel bore 26 forward of chamber 16 or, if the macroprojectile is slightly oversized for the barrel bore, at the bore end of the chamber 16. Chamber 16, being dimensioned for a 5.59mm (22 cal.) long rifle cartridge, is able to accept both the macroprojectile and the blank cartridge. The 5.59mm (.22 cal.) blank cartridge 24 is then inserted into chamber 16. The bolt apparatus designated generally at 18 is then placed on top of the barrel and ram 20 thrust downwards such that firing pin 22 hits the rim of the cartridge 24 and fires it. A vacuum is pulled in the vacuum chamber during the firing process, and generally the vacuum provided by vacuum means 40 should be within the range of from about 635.0 to 736.6mm (25 to 29 inches) of mercury, i.e. a light vacuum. When the blank cartridge is fired the macroprojectile 42 is propelled out of the barrel at muzzle end 14.

The macroprojectile 42 comes out of muzzle end 14 and almost immediately impacts against stopping plate 46. The microprojectiles, carrying the biological material, pass through orifice 48, forwardly through the frustoconical bore in stopping plate 46, and in shotgun pattern impinge against target cells in petri dish 36. In this manner a host of target cells are simultaneously impregnated with the DNA material.

As best illustrated in Figures 4 and 6, the stopping plate holder shelf has a cylindrical recess 58 into which cushion ring 39 and stopping plate support 40A are inserted. Topping these, metal stopping plate holder 50 is affixed to stopping plate holder shelf 38 so that annular projection 60 on the underside of stopping plate holder 50 is concentrically seated in recess 58. Stopping plate 46 is then inserted into opening 62 in stopping plate holder 50. Care is taken that stopping plate holder shelf 38, stopping plate holder 50 and stopping plate 46 are dimensioned and positioned such that orifice 48 is coaxial with barrel 26.

In prior art particle guns, several problems have existed which cause a large incidence of failure. The prior art particle gun is subject to large variation and debris "blow by" and variation of velocity from macroprojectile to macroprojectile due to differences in diameter. Differences in diameter of the macroprojectile as small as 0.0127mm (0.0005 inches) result in lowered velocity (undersized) or inability to place the macroprojectile in the bore of the barrel (Figure 3, 26) (oversized). These problems have been solved in the present invention by making chamber 16 of 5.59mm (.22 cal.) long rifle length and by making the bore 26 a rifled bore as shown in Figure 5 at 52 and 54.

The 5.59mm (.22 cal.) long rifle bore allows many sizes of macroprojectiles between 5.537 mm and 5.639 mm (.218 inches and 0.222) inches to be easily accommodated in the chamber along with the blank cartridge. When the smooth bore of the prior art particle guns is replaced with the rifle bore of the present gun, preferably having a quarter twist for every 152.4mm (six inches), it is found that there is an effective seal presented between the macroprojectile and the bore 26. The result is that macroprojectiles of varying sizes produced by normal manufacturing methods can be employed and remain effectively sealed in the bore, maintaining consistent velocity from firing to firing. Thus the risk of cell damage from "blow by" is substantially reduced. Moreover, the risk of complete failure of undersized macroprojectiles to pass through the barrel (because of extreme blow by) is removed. In addition, the barrel is effectively-cleaned during each firing, so that particulates which precede the macro and microprojectiles are unlikely to occur. This result is surprising because one would ordinarily expect that rifling might cause abrasion to the macroprojectile, resulting in additional debris. Spin imparted to the macroprojectile by the rifling might also be expected to cause loss of the microprojectile and associated biological material from the tip of the macroprojectile; however, this does not occur.

Another important improvement of the present invention is the composition of the macroprojectile. In the prior art, the macroprojectile has been made from somewhat resilient thermoplastic polymeric materials, particularly ultra high molecular weight polyethylene, which has contributed to a high level of failures. In particular, the impact caused by firing of the cartridge has in some instances caused the macroprojectile to deteriorate upon impact with the stopping plate, resulting in extrusion of portions of the macroprojectile through the stopping plate. This produces particulate debris which interferes with the successful introduction of the biological material into the cells by damaging the cells and can actually drive the cells out of petri dish 36 or even damage the dish itself. It has been found, however, that if the prior art UHMWPE material is replaced with a harder material such as acetal, preferably that sold under the trademark POLYPENCO™, available from Westlakes Plastics Company of Lenni, Pennsylvania 19052, the risk of failures is substantially decreased, yet shattering the macroprojectile or the stopping plate does not occur. In addition, this selection of materials not only reduces the failure rate of the gun, it surprisingly increases the numbers of transformed cells in successful bombardments.

The following examples are offered to further illustrate the apparatus and methods of the present invention.

EXAMPLE I

The performance of the smooth bore and rifled bore barrels was compared using a gene for the enzyme beta glucuronidase (GUS) gene in black Mexican sweet (BMS) maize tissue culture cells. The DNA was constructed such that it was capable of functioning in plant cells and could be detected by a well known cytochemical staining procedure. The DNA was mixed with beads as indicated previously. BMS suspension cells of maize were prepared for the particle gun by subdividing the suspension culture cells into 100mg aliquots on filter paper in 3.3cm petri plates. Each treatment unit consisted of a petri dish with 100mg of maize cells. Following particle gun bombardment, cells were incubated for 48 hours and stained by addition of 5-Bromo-4-Chloro-3-Indolyl-Beta-D Glucuronide (X-Glu). The results of this experiment are shown in Table 1. The smooth bore barrel had a 30% failure rate, i.e. failure to produce transient expression of the beta glucuronidase gene in the bombarded cells. Twenty percent of the samples had velocity which was so low that there were no stained cells, while an additional 10% showed sample damage in which some cells were blown off the filter paper and a hole was observed in the filter paper as well. The rifled bore barrel had a zero failure rate. In addition, in comparing only those samples in which transient expression of the beta glucuronidase gene was observed, the rifle bore barrel produced approximately 15% more stained cells per plate than the smooth bore barrel.

TABLE 1

Comparison of Smooth Bore and Rifled Barrel Failure Rate and
Transient Gene Expression Using a Cytochemical Stain for
Expression of the Beta Glucuronidase Gene (GUS) in
Black Mexican Suspension Cells of Maize

| Barrel Type | Proportion of Failure | | Number Samples | Mean Number Expressing Cells |
|---|---|---|---|---|
| | Low Velocity | Sample Damage | | |
| Smooth | 20% | 10% | 10 | 130.6 |
| Rifled | 0% | 0% | 10 | 148.7 |

## EXAMPLE II

Two macroprojectile materials, ultra high molecular weight polyethylene and acetal, were compared using the GUS gene in transient expression experiments using maize suspension culture cells and tobacco suspension culture cells. The number of stained cells was measured as in the previous experiments. Results are shown in Table 2. Summarized over the six separate experiments, the acetal macroprojectile produces 70% more stained cells than the ultra high molecular weight polyethylone macroprojectile.

TABLE 2

Transient Gene Expression of
Ultra High Molecular Weight Polyethelene and

Acetal Microprojectiles Using the
GUS Gene over Several Experiments

| Experiment Number | Species | N | UHMW-P Total Stained Cells | N | Acetal Total Stained Cells |
|---|---|---|---|---|---|
| 1 | Maize | 4 | 186 | 5 | 338 |
| 2 | Maize | 9 | 315 | 10 | 485 |
| 3 | Maize | 5 | 52 | 5 | 65 |
| 4 | Maize | 6 | 50 | 4 | 64 |
| 5 | Tobacco | 5 | 122 | 2 | 143 |
| 6 | Tobacco | 5 | 64 | 8 | 221 |
| Summary | | 34 | 789 | 34 | 1317 |
| Mean | | | 131.5 | | 219.5 |

## EXAMPLE III

The method of combining tungsten beads and DNA was altered in another series of experiments using two polyamines, spermidine and spermine. As shown in Table 3, the use of spermine produced 2.5 times more transformed cells than spermidine.

5

TABLE 3

Comparison of Transient GUS Gene Expression
Between Spermidine and Spermine Polyamines in
DNA/Bead Mixtures in Tobacco and Maize Suspension Cultures

| Species | N | Mean Number Stained Cells | |
| --- | --- | --- | --- |
| | | Spermidine | Spermine |
| Tobacco | 28 | 20.6 | 69.9 |
| Maize | 20 | 42.4 | 113.2 |

## Claims

1. A particle gun for transporting biological materials into living cells, comprising:
   means for holding living cells (56);
   a barrel (12) aimed at the cell holding means and comprising
   a cartridge chamber (16) at one end for receipt of a macroprojectile carrying biological material and a firing cartridge containing a charge sufficient, when the cartridge is fired, to propel the biological material into the interior of the living cells,
   a rifled bore (52,54), and
   a muzzle (14) at the other end of the barrel;
   firing pin means (18) associated with the cartridge chamber;
   a stopping plate (46) positioned between the barrel and the cell holding means for stopping the macroprojectile, the stopping plate having an orifice (48) which allows the biological material carried on the macroprojectile to pass therethrough; and
   a vacuum chamber (30) surrounding the muzzle end of the barrel, the stopping plate, and the cell holding means.

2. The particle gun of claim 1 wherein the rifling has a twist over the barrel length.

3. The particle gun of claim 1 or 2 wherein the barrel has a vent proximal to its muzzle end for gas and debris release.

4. The particle gun according to any one of the preceding claims wherein the barrel has an elongated cartridge chamber in excess of the length of the cartridge used in firing.

5. The particle gun of Claim 4 wherein the barrel has a 5.59mm (.22 cal.) long rifle chamber.

6. The particle gun of any one of the preceding claims wherein the macroprojectile is a polymeric plastic material capable of withstanding impact with the stopping plate without forming debris.

7. The particle gun of Claim 6 wherein the macroprojectile is an acetal plastic.

8. A method of transferring biological materials into the cytoplasm of living cells without significant debris also being transferred into the cells, comprising; firing a macroprojectile carrying the desired biological material at the cells through a barrel comprising a rifled bore and causing the biological material to leave the macroprojectile at a velocity sufficient to penetrate the cells.

9. The method of Claim 8 wherein the biological materials are carried on the macroprojectile by microprojectiles consisting of biologically inert, high mass metal beads having a particle size of from about 1.0 μm to about 4.0 μm.

10. The method of Claim 9 wherein the microprojectiles have a particle size of from about 1.0 μm to about 1.5 μm.

**11.** The method of Claim 9 or 10 wherein the inert metal is selected from tungsten, palladium, iridium, platinum and gold.

**12.** The method of Claim 11 wherein the metal is tungsten.

**13.** The method of any one of claims 9-12 wherein the microprojectiles are mixed with the biological material and sufficient polyamine to enhance adherence of the biological material to the beads.

**14.** The method of Claim 13 wherein the polyamine is selected from spermine, caldine, and thermine.

**15.** The method according to Claim 14 wherein the polyamine is spermine.

**Patentansprüche**

**1.** Ein Partikelgewehr zum Transportieren von biologischen Materialien in lebende Zellen, umfassend:
ein Mittel zum Halten lebender Zellen (56);
einen Lauf (12), der auf das Zellenhaltemittel gerichtet ist, umfassend:
eine Patronenkammer (16) an einem Ende zur Aufnahme eines biologisches Material tragenden Makroprojektils und einer Schießpatrone, die eine Ladung enthält, welche, wenn die Patrone abgefeuert wird, ausreichend ist, das biologische Material in das Innere der lebenden Zellen zu treiben,
eine gezogene Laufseele (52, 54), und
eine Mündung (14) an dem anderen Ende des Laufs;
ein Zündstiftmittel (18), das mit der Patronenkammer verbunden ist;
ein Stoppplatte (46), die zwischen dem Lauf und dem Zellenhaltemittel zum Stoppen des Makroprojektils positioniert ist, wobei die Stoppplatte eine Öffnung (48) hat, welche es dem auf dem Makroprojektil getragenen biologischen Material ermöglicht, dadurch hindurchzugehen; und
eine Vakuumkammer (30), welche das Mündungsende des Laufs, die Stoppplatte und das Zellenhaltemittel umgibt.

**2.** Das Partikelgewehr des Anspruchs 1, worin die Riefung eine Verdrillung über die Lauflänge hat.

**3.** Das Partikelgewehr des Anspruchs 1 oder 2, worin der Lauf proximal zu seinem Mündungsende eine Abzugsöffnung für Gas- und Überbleibselfreigabe hat.

**4.** Das Partikelgewehr gemäß irgendeinem der vorhergehenden Ansprüche, worin der Lauf eine langgestreckte Patronenkammer über die Länge der beim Abfeuern verwendeten Patrone hinaus hat.

**5.** Das Partikelgewehr des Anspruchs 4, worin der Lauf eine 5,59 mm (0,22 cal.) lange Gewehrkammer hat.

**6.** Das Partikelgewehr von irgendeinem der vorhergehenden Ansprüche, worin das Makroprojektil ein polymeres Kunststoffmaterial ist, welches fähig ist, dem Zusammenstoß mit der Stoppplatte ohne Bilden von Überbleibseln standzuhalten.

**7.** Das Partikelgewehr des Anspruchs 6, worin das Makroprojektil ein Azetalkunststoff ist.

**8.** Ein Verfahren zum Übertragen biologischer Materialien in das Zytoplasma von lebenden Zellen, ohne daß signifikante Überbleibsel auch in die Zellen übertragen werden, umfassend: Abfeuern eines Makroprojektils, welches das in den Zellen gewünschte biologische Material trägt, durch einen Lauf, der eine gezogene Laufseele umfaßt, und Bewirken, daß das biologische Material das Makroprojektil bei einer Geschwindigkeit verläßt, die zum Durchdringen der Zellen ausreichend ist.

**9.** Das Verfahren des Anspruchs 8, worin die biologischen Materialien auf dem Makroprojektil durch Mikroprojektile, die aus biologisch inerten Metallkügelchen hoher Masse bestehen, welche eine Partikelgröße von etwa 1,0 µm bis etwa 4,0 µm haben.

**10.** Das Verfahren des Anspruchs 9, worin die Mikroprojektile eine Partikelgröße von etwa 1,0 µm bis etwa 1,5 µm haben.

**11.** Das Verfahren des Anspruchs 9 oder 10, worin das inerte Metall ausgewählt ist aus Wolfram, Palladium, Iridium, Platin und Gold.

12. Das Verfahren des Anspruchs 11, worin das Metall Wolfram ist.

13. Das Verfahren von irgendeinem der Ansprüche 9-12, worin die Mikroprojektile mit dem biologischen Material und genügend Polyamin zur Erhöhung der Haftung des biologischen Materials an den Kügelchen gemischt sind.

14. Das Verfahren des Anspruchs 13, worin das Polyamin ausgewählt ist aus Spermin, Caldin und Thermin.

15. Das Verfahren gemäß Anspruch 14, worin das Polyamin Spermin ist.


## Revendications

1. Canon à particules pour transférer des substances biologiques dans des cellules vivantes, comportant :
   des moyens pour supporter des cellules vivantes (56) ;
   un fût (12) pointé sur les moyens supportant les cellules et comportant
   une chambre de cartouche (16) à une extrémité pour la réception d'un macroprojectile portant la substance biologique et une cartouche de mise à feu contenant une charge suffisante, lorsque la cartouche est mise à feu, pour propulser la substance biologique dans l'intérieur des cellules vivantes,
   un alésage rayé (52, 54), et
   une gueule (14) à l'autre extrémité du fût;
   des moyens formant percuteur (18) associés à la chambre de cartouche ;
   une plaque d'arrêt (46) disposée entre le fût et les moyens supportant les cellules pour arrêter le macro-projectile, la plaque d'arrêt possédant un orifice (48) permettant à la substance biologique portée par le macro-projectile de le traverser ; et
   une chambre à vide (30) entourant la gueule du fût, la plaque d'arrêt, et les moyens supportant les cellu- les.

2. Canon à particules selon la revendication 1, dans lequel les rayures forment une torsade sur la longueur du fût.

3. Canon à particules selon la revendication 1 ou 2, dans lequel le fût possède un orifice à proximité de sa gueule pour un dégagement des gaz et des débris.

4. Canon à particules selon l'une quelconque des revendications précédentes, dans lequel le fût possède une chambre de cartouche allongée excédant la longueur de la cartouche utilisée durant le tir.

5. Canon à particules selon la revendication 4, dans lequel le fût possède une chambre rayée allongée de 5,59 mm (calibre 22).

6. Canon à particules selon l'une quelconque des revendications précédentes, dans lequel le macroprojectile est une matière plastique polymère pouvant résister aux chocs avec la plaque d'arrêt sans former de débris.

7. Canon à particules selon la revendication 6, dans lequel le macroprojectile est une matière plastique acétal.

8. Procédé de transfert de substances biologiques dans le cytoplasme de cellules vivantes sans que des débris importants soient également transférés dans les cellules, consistant à un macroprojectile portant la substance biologique désirée aux cellules à travers un fût comportant un alésage rayé et provoquer la libération de la substance biologique du macroprojectile à une vitesse suffisante pour pénétrer dans les cellules.

9. Procédé selon la revendication 8, dans lequel les substances biologiques sont portées sur le macroprojectile par des microprojectiles consistant en des perles de métal de masse élevée, biologiquement inerte, possédant une dimension de particule d'environ 1,0 μm à environ 4,0 μm.

10. Procédé selon la revendication 9, dans lequel les microprojectiles possèdent une dimension de particule d'environ 1,0 μm à environ 1,5 μm.

11. Procédé selon la revendication 9 ou 10, dans lequel le métal inerte est choisi parmi le tungstène, le palladium, l'iridium, le platine et l'or.

12. Procédé selon la revendication 11, dans lequel le métal est du tungstène.

13. Procédé selon l'une quelconque des revendications 9 à 12, dans lequel les microprojectiles sont mélangés à la substance biologique et à une quantité suffisante de polyamine pour accroître l'adhérence de la substance biologique aux perles.

14. Procédé selon la revendication 13, dans lequel la polyamine est choisie parmi la spermine, la caldine et la thermine.

15. Procédé selon la revendication 14, dans lequel la polyamine est de la spermine.

Fig. 1

Fig. 2

Fig. 3

48
46
50
62
6
6

50
62
60

Fig.6

40A
39

38
58

Fig.4

28
12
26
52
54
28

Fig.5